Europäisches Patentamt

European Patent Office

Office européen des brevets

(11) Publication number: **0 326 148**
A1

# (12) EUROPEAN PATENT APPLICATION

(21) Application number: **89101423.5**

(22) Date of filing: **27.01.89**

(51) Int. Cl.⁴ **A61K 39/40 , A61K 39/104 , A61K 39/108 , A61K 39/112 , C12P 21/00 , C12N 5/00**

(30) Priority: **29.01.88 JP 20907/88**

(43) Date of publication of application:
**02.08.89 Bulletin 89/31**

(84) Designated Contracting States:
**DE NL SE**

(71) Applicant: **SUMITOMO CHEMICAL COMPANY, LIMITED**
**Kitahama 4-chome 5-33**
**Chuo-ku Osaka 541(JP)**

Applicant: **SUMITOMO PHARMACEUTICALS COMPANY, LIMITED**
**40, Dosho-machi 2-chome**
**Higashi-ku Osaka-shi Osaka-fu(JP)**

(72) Inventor: **Ohtsuka, Hiroshi**
**16-40-601, Takagihigashi-machi**
**Nishinomiya Hyogo(JP)**
Inventor: **Ochi, Hiroshi**
**2-11-8-110, Sonehigashi-machi**
**Toyonaka Osaka(JP)**
Inventor: **Higuchi, Atsuko**
**2-1-211, Komyo-cho Takarazuka**

**Hyogo(JP)**
Inventor: **Yokota, Shinichi**
**2-14-7, Mefu Takarazuka**
**Hyogo(JP)**
Inventor: **Noguchi, Hiroshi**
**4-4-153, Seiwadainishi**
**Kawanishi Hyogo(JP)**
Inventor: **Terashima, Masazumi**
**2-29-7, Ohike Ibaraki**
**Osaka(JP)**
Inventor: **Uezumi, Ikuko**
**5-1-30, Shuntoku-cho Higashiosaka**
**Osaka(JP)**
Inventor: **Kato, Masuhiro**
**2-10-2-235, Sonehigashi-machi**
**Toyonaka Osaka(JP)**
Inventor: **Okuda, Takao**
**4-4-22-802, Kamishinden Toyonaka**
**Osaka(JP)**

(74) Representative: **Kolb, Helga, Dr. Dipl.-Chem. et al**
**Hoffmann, Eitle & Partner Patentanwälte**
**Arabellastrasse 4**
**D-8000 München 81(DE)**

(54) **Human monoclonal antibody, its production and use as preventive or therapeutic agent, and hybrid cell lines.**

(57) A human monoclonal antibody having a binding property to the lipopolysaccharides of the strains of Pseudomonas aeruginosa classified to Serotypes A, F, G and M under the Japanese Commitee's Classification and also a binding property to the lipid A of Gram-negative bacteria such as Escherichia, Salmonella and Pseudomonas, which is effective in prevention and treatment of infections caused by bacterial comprising Pseudomonas aeruginosa and endotoxin shock caused by Gram-negative bacteria and its production and use as preventive or therapeutic agent, and hybrid cell lines are disclosed.

EP 0 326 148 A1

## HUMAN MONOCLONAL ANTIBODY, ITS PRODUCTION AND USE AS PREVENTIVE OR THERAPEUTIC AGENT, AND HYBRID CELL LINES

The present invention relates to a human monoclonal antibody to Pseudomonas aeruginosa (hereinafter referred to as "P. aeruginosa" or "PA"), and its production and use. More particularly, it relates to a human monoclonal antibody, which can recognize a structure common to the lipopolysaccharides in P. aeruginosa of different serotypes and shows a binding property to P. aeruginosa of two or more serotypes, and its production and use.

The human monoclonal antibody according to the invention shows, as stated above, a binding property to P. aeruginosa of two or more serotypes and also to the lipid A of Gram-negative bacteria, particularly bacteria belonging to Escherichia, Salmonella and Pseudomonas, so that it is useful for prevention and treatment of infectious diseases caused by P. aeruginosa and also for endotoxin shocks caused by Gram-negative bacteria.

Bacteria causing infectious diseases, i.e. prophlogistic bacteria, are varied with development and change of antibiotics as clinically used. As the result, infectious diseases with bacteria originally having only low pathogenicity or virulence may increase. Thus, P. aeruginosa is currently one of the major pathogenic bacteria causing infectious diseases, of which serious symptoms often lead patients to death, particularly when their immunological competence is low due to continuous administration of immunosupressants, suffering from cancer or burn or the like.

Among various preventive or therapeutic methods for bacterial infections, the most prevailing one is chemotherapy by the use of antibiotics or antimicrobial agents. In fact, there have been developed various antibiotics including streptomycin, kanamycin, penicillin, cephalosporin, etc., which are sensitive to almost all Gram-positive bacteria (e.g. Staphylococci) and Gram-negative bacteria (e.g. E. coli) and produce a prominent clinical effect. However, there are known only few medicinal products sensitive to P. aeruginosa. Even those medicianl products act on P. aeruginosa only bacteriostatically and not bacteriocidally. Thus, they can prevent the growth of P. aeruginosa but do not clinically exhibit any remarkable therapeutic effect.

The other preventive or therapeutic method is antibody therapy comprising administration of immunoglobulin. This method is often performed in association with chemotherapy and nowdays attracts much attention as a substitute for chemotherapy. A serum of high antibody titer can be obtained by active immunization of animals such as horse or rabbit, and antibody therapy can be made by administration of such serum. In fact, its remarkable therapeutic effect was proved on experimental infections using various animals. It is known from the cases of diphtheria toxin and viper toxin that antibody therapy using sera originated from animals is quite effective even on human beings. However, introduction of a heterogenous protein obtained from animals into a human body causes such a serious side-effect as anaphylaxis or any other allergic reaction. It is thus highly desired to develop human immunoglobulin having a high antibody titer against bacteria and showing a prominent therapeutic effect on bacterial infections.

Conventional human immunoglobulin preparations are manufactured by collecting blood from healthy persons or bacteria-infected patients, subjecting the blood to fractionation to obtain an immunoglobulin fraction, purifying the immunoglobulin fraction and eliminating agglutinating materials therefrom by addition of ethylene glycol, treatment with protease, sulfonization, DEAE-column chromatography, etc., followed by formulation of the resulting product into intramuscularly or intravenously injectionable preparations. These preparations are advantageous in not causing anaphylaxis or any other side-effect as seen on administration of immunoglobulin originated from animals but have some drawbacks. One of such drawbacks is that their antibody titer against bacteria is low so that a sufficient therapeutic effect can not necessarily be produced. Another drawback is that their stable supply with a high antibody titer in a large amount is difficult, because they are manufactured using the blood collected from healthy persons or bacteria-infected patients and the constant and continuous obtainment of sera having a high antibody titer is quite hard. A further drawback is that they may be contaminated with hepatitis virus (e.g. HB virus), Adult T cell leukaemia virus (ATLV, HTLV), etc., because the blood as the starting mterial is obtained from a number of unknown persons. In order to overcome these drawbacks, production of a human monoclonal antibody having a strongly inhibitive effect on the infections with P. aeruginosa is highly desirable.

When an antibody is bound to the surface layer of a bactrial body, the phagocytosis of a macrophase on the bacterial body is accelerated (i.e. acceleration of phagocytosis due to opsonization), or the lysis of the bacterial body by a complement takes place. As the target antigen at the surface layer of the bacterial body of P. aeruginosa, there are known lipopolysaccharide (LPS), envelope protein, flagellum, pilus, etc. Among them, the O-polysaccharide which is a constituent of LPS, located at the most outside of the surface layer and designated as an "O-antigen", has a strong antigenicity, and the antibody corresponding to the O-

EP 0 326 148 A1

antigen exhibits generally a high preventive or therapeutic effect.

The O-antigen is diversified in its chemical structure so that the strains belonging to P. aeruginosa are classified on the difference in immunological reactivity with an anti-serum or a mouse monoclonal antibody as prepared to the O-antigen of the standard strain of P. aeruginosa. This classification is known as a serotype, and typical examples of the serotype classification are as follows: Types 1 to 17 according to the classification by Homma et al. (Japan.J.Exp.Med., 44, 1, (1974)); Types 1 to 7 according to the classification by Fisher et al (J.Bacteriol., 98, 835 (1969)); Types A to M according to the classification by Nippon Ryokunoh-kin Kenkyukai Kesseigata-betsu Kento linkai (Committee of Study on Serotype Classification, Japanese Study Group on Pseudomonas aeruginosa (hereinafter referred to as "Japanese Committee") (Japan.J.Exp.Med., 45, 329 (1976)); Types 1 to 17 according to the classification by International Antigenic Typing System (IATS), etc. These classifications and their cross-relations are shown in Table 1 (Japan.J.Exp.Med., 46, 329 (1976)).

Table 1

| Serotype classification of P. aeruginosa | | | |
|---|---|---|---|
| Japanese Commitee 1976 | Homma et al 1974 | ITATS 1983 | Fisher et al 1969 |
| A | 1 | 3 | - |
| B | 2, 7, 13, 16 | 2, 5, 16 | 3, 7 |
| C | 3 | 8 | 6 |
| D | 4 | 9 | - |
| E | 5 | 11 | 2 |
| F | 6 | 4 | - |
| G | 8 | 6 | 1 |
| H | 9 | 10 | 5 |
| I | 10 | 1 | 4 |
| J | 11 | 15 | - |
| K | 12 | 13 | - |
| L | 14 | - | - |
| M | 15, 17 | - | - |
| - | - | 7, 12, 14, 17 | - |

P. aeruginosa O-antigen has a polymeric structure comprising repeating units of 2 to 5 saccharides, and the sequence of such repeating units is varied with each serotype. It is known that the antibody specific to a certain O-antigen shows a strong preventive or therapeutic effect for infections against P. aeruginosa of the serotype to which said O-antigen belongs but does not show any effect against P. aeruginosa of any other serotype. It is also known that a human monoclonal antibody can be produced continuously by the use of an established cell line capable of producing a specific antibody.

Establishment of a cell line capable of producing a human monoclonal antibody specific to the O-antigen of the lipopolysaccharide of P. aeruginosa can be made by application of an EB (Epstein-Barr) virus transformation method, a cell fusion method and the like. For instance, JP-A1-61-69796 discloses that transformation of spleen lymphocytes obtained from patients infected with P. aeruginosa in the past with EB virus affords EB virus-transformed cells capable of producing a human monoclonal antibody specific to the O-antigen of P. aeruginosa. JP-A1-61-152281 also discloses that stimulation of tonsil cells obtained from patients of tonsillitis with poke-weed mitogen (PWM) and fusion of the stimulated tonsil cells with mouse myeloma cells (P3-X63-Ag8-UI strain) give human-mouse hybridomas capable of producing a human monoclonal antibody specific to the O-antigen of P. aeruginosa. However, the human monoclonal antibodies as disclosed above are restricted to serotype-specific human monoclonal antibodies, i.e. having a binding property to P. aeruginosa of a certain serotype and having no binding property to P. aeruginosa of other serotypes.

On the other hand, JP-A1-62-187417 discloses a monoclonal antibody cross-binding and cross-preventive to P. aeruginosa of two or more serotypes, which is based on obtainment of a cell capable of producing a monoclonal antibody cross-binding to the strains of Types 2, 5 and 16 under the IATS's Classiffication as well as the strains of Types 3 and 7 under the Fisher's Classification. As seen in Table 1 above, however, all these strains fall in Type B under the Japanese Commitee's Classification, and

3

therefore said monoclonal antibody still remains specific to the strains of a single serotype. Thus, said monoclonal antibody shows a binding property to only less than 20 % of clinical isolate strains, and therefore plural numbers of serotype specific monoclonal antibodies are required for the practical use.

Progress of the infectious diseases caused by Gram-negative bacteria results in septicemia, whereby the patients' life falls frequently in danger. Typical symptoms of septicemia are pyrexia, rigor, organic insufficiency, shock, etc. Septic shock is called "endotoxin shock" and caused by LPS (lipopolysaccharide), which is known as endotoxin and released from the bacterial body into the human body as the result of proliferation and death of a large number of Gram-negative bacteria and/or disruption of said bacteria due to the action of antibiotics as given. The active site causing endotoxin shock is known to be lipid A in LPS. However, any effective preventive or therapeutic method for endotoxin shock is presently not available, and the mortality due to exdotoxin shock is so high as 50 %. Because of this reason, a strong demand for development of an effective preventive or therapeutic method has been present.

Ziegler et al. administered a heat-sterilized E. coli J5 (Rc-type rough mutant) as a vaccine to healthy persons to obtain an anti-serum, which was demonstrated to be effective in prevention of fatal shock due to bacteremia (New England J. Med., 307, 1225 - 1230, (1982)).

Subsequently, various animal and clinical studies were made, and it was revealed that various anti-sera and monoclonal antibodies specific to E. coli J5 or the core glycolipid of Gram-negative bacteria are effective in prevention of endotoxin shock (WO 8404458; WO 8501659; EP-A-0174204; JA-A-61-130300; Mutharia et al.: Inf.Immun., 45, 631 - 636 (1984); Teng et al: Proc. Natl. Acad. Sci., USA, 82, 1790 - 1794 (1985); Gigliotti & Shenep: J.Inf.Dis., 151, 1005 - 1011 (1985); Braude et al: J.Inf.Dis., 136 (Suppl), S167 - 173 (1977); Nellesand Niswander: Inf.Immun., 46, 677 - 681 (1984); Pollack et al: J. Clin. Invest., 72, 1874 - 1881 (1983); Young et al: Clin. Res., 30, 522A (1982); Young et al: Clin. Res., 32, 518A (1984)).

As explained above, the known antibody specific to the O-antigen of the P. aeruginosa lipopolysaccharide is effective only in prevention and treatment of the infections caused by P. aeruginosa of the serotype to which said O-antigen belongs and does not produce any preventive or therapeutic effect for the infections caused by P. aeruginosa of any other serotype. Accordingly, it is necessary for prevention or treatment of the infections with P. aeruginosa of any serotype to provide at least 13 to 17 kinds of monoclonal antiboides specific to the entire serotypes of P. aeruginosa. For this purpose, cell lines producing human monoclonal antibodies specific to at least 13 to 17 kinds of serotypes must be established. Further, cultivation of each cell line in a large scale as well as purification of the produced antibody is required, and this creates a great problem from the industrial viewpoint. Development of a human monoclonal antibody which can be bound not to only one serotype strain of P. aeruginosa but to plural or all serotype strains so as to produce a preventive or therapeutic effect on the infections due to P. aeruginosa of various serotypes is highly desired.

As the result of an extensive study to obtain a human monoclonal antibody effective in prevention and treatment of the infectious disease caused by P. aeruginosa and a high titer human immunoglobulin containing the same at an industrial scale, it has now been succeeded in establishing a cell line producing a human monoclonal antibody which specifically binds to the common chemical structure to LPS in P. aeruginosa of different serotypes from human B lymphocytes. By the cultivation of such cell line, a human monoclonal antibody being reactive to two or more kinds of LPS of different serotypes and having a preventive or therapeutic effect for the infections with P. aeruginosa of two or more serotypes is obtainable. More specifically, the monoclonal antibody according to the invention is characteristic in showing a binding property specific to almost all strains of P. aeruginosa belonging to Types A, F, G and M under the Japanese Committee's Classification and therefore can be distinguished clearly from conventional monoclonal antibodies specific to only one serotype strain as well as the known monoclonal antibody cross-binding to strains of serotypes within one sub-group under said Classification as disclosed in JP-A1-62-187417.

Accordingly, a basic object of the present invention is to provide a human antibody which can recognize an antigen-determinating site common to the LPS of P. aeruginosa, particularly a human monoclonal antibody having a single antigen-specificity (monospecific antibody). Another object of the invention is to provide a human cell line which is capable of producing said human antibody continuously. A further object of the invention is to provide a human immunoglobulin preparation for prevention or treatment of infections with P. aeruginosa comprising said human antibody. A further object of the invention is to provide a process for production of said human antibody by in vitro or in vivo cultivation of said human cell line. A still further object of the invention is to provide a method for obtaining said human cell line. A still further object of the invention is to provide a novel antibody which can recognize the chemical structure common to the LPS in P. aeruginosa and is cross-reactive to the lipid A originated from Gram-negative bacteria such as P. aeruginosa, E. coli and S. thyphmurium. These and other objections of the invention will be apparent to those skilled in the art from the foregoing and subsequent descriptions in the present specification.

The human monoclonal antibody according to the invention, which is specific to the antigen-determinating site common to the LPS in P. aeruginosa, is intended to mean a single human monoclonal antibody which can recognize the antigen-determinating site common to the LPS of P. aeruginosa of two or more serotypes, e.g. of Types A, G, F and M, and is cross-reactive to the lipid A in the LPS originated from Gram-negative bacteria. It can be produced from a single antibody-producing clone.

Said human monoclonal antibody has a binding property (binding activity) to strains of P. aeruginosa of two or more serotypes or the LPS derived therefrom, i.e. a capability of accelerating a bacteriocidal property in the presence of a complement as well as a phagocytosis by a neutrophile and macrophage (opsonization), and can prevent and treat the infectious diseases with P. aeruginosa. The epitopes of said human monoclonal antibody are present in the LPS of the strains of Types A, G, F and M, specifically at the lipid A site and the polysaccharide portion in LPS.

The serotype used herein is in accordance with the Classification pursuant to the Japanese Commitee, which is determined on the difference in immunochemical reaction using an anti-serum or a mouse monoclonal antibody specifically reactive to the standard strain of P. aeruginosa of each serotype.

The LPS stands for a lipopolysaccharide which is a constituent of the surface layer of a Gram-negative bacterial body and comprises a lipid (lipid A) and a core portion bonded thereto, said core portion comprising 2-keto-3-deoxyoctonic acid, heptose, phosphoric acid, etc. as its constituents and bearing a polysaccharide side chain called "O-antigen" at its end. The O-antigen has a polymeric structure comprising a straight chain consisting of 2 to 5 monosaccharides as the repeating unit. The composition, arrangement and configuration of the saccharide chain are different among serotypes, and this difference is the base for classification with serotypes.

As stated above, the present invention provides a human monoclonal antibody, which can recognize the common antigen-determinating site in the LPS of P. aeruginosa and also cross-react with lipid A originating from Gram-negative bacteria.

The human monoclonal antibody cross-reactive to lipid A originating from Gram-negative bacteria is a single human-type antibody which can specifically recognize lipid A isolated and purified from Gram-negative bacteria such as E. coli, S. typhimurium and P. aeruginosa. Since the human monoclonal antibody has a binding property (binding activity) specifically to lipid A of Gram-negative bacteria including P. aeruginosa and a neutralizing property (neutralizing activity) of the biological activities such as mitogen activity, fatal toxicity and Schwarzman reaction as caused by lipid A in addition to said reactivity to the common antigen-determinating site in the LPS of P. aeruginosa, it is useful as a preventive or therapeutic agent for endotoxin shock caused by Gram-negative bacteria.

The human monoclonal antibody can also show a binding property to LPS liberated from E. coli or S. typhimurium or to the LPS portion in case of the lipid A site being exposed as in a strain deficient in O-antigen and core polysaccharide (e.g. deep rough type strain).

Lipid A constitutes a glycolipid portion in LPS and can be prepared from LPS by cleavage of the ketoside bond in the ketodeoxyoctonic acid (KDO) with an acid. It comprises a skeleton of beta(1-6)-disaccharide 1,4ʹ-diphosphoric ester and fatty acids attached thereto, the amount of the fatty acids being 4 mol per 1 mol of Lipid A. The kind, number and bonding position of the fatty acids are varied with each strain.

The human cell line which can continuously produce the human monoclonal antibody of the invention, which is specifically reactive to the common antigen-determinating site in LPS of P. aeruginosa and particularly cross-reactive to lipid A of Gram-negative bacteria, may be produced by various procedures, of which some typical examples are shown below.

In the first procedure, human B lymphocytes sensitized (immunized) with P. aeruginosa (live bacteria or killed bacteria with formalin or heating), LPS originated from P. aeruginosa or Gram-negative bacteria which has a lipid A structure on its surface (live bacteria or killed bacteria with formalin or heating), preferably E. coli J5 or S. typhimurium Rc, Rd or Re mutant or LPS or lipid A originated from those strains, in vivo or in vitro are infected with EB (Epstein-Barr) virus by mixing, whereby said B lymphocytes are transformed to the ones which can proliferate continuously. From the thus transformed cells (before or after cloning), the ones producing an antibody having a reactive specificity are selected and proliferated continuously in vitro so as to establish cell lines which can produce said antibody continuously. For the selection, there may be used ELISA (enzyme-linked immunosorbent assay) with a bacterial body screening panel comprising P. aeruginosa strains of 17 kinds different in serotype and E. coli O-111:B4 and J5. Selection may be also made by ELISA using LPS and lipid A originated from P. aeruginosa, E. coli O-111:B4 and J5 and S. typhimurium wild strain and Ra to Re mutants as the antigens.

In the second procedure, human B lymphocytes sensitized with P. aeruginosa (live bacteria or killed

5

bacteria with formalin or heating), LPS originated from P. aeruginosa or Gram-negative bacteria which has a lipid A structure on its surface (live bacteria or killed bacteria with formalin or heating), preferably E. coli J5 or S. typhimurium Rc, Rd or Re mutant or LPS or lipid A originated from those strains, are subjected to cell fusion with myeloma cells or B lymphoblastoid cells to establish cell lines which can be proliferated in vitro continuously and produce an LPS-specific antibody continuously.

In the third procedure, the EB virus-transformed cells as established in the first procedure are subjected to cell fusion with myeloma cells or B lymphoblastoid cells to establish a cell line which can be proliferated in vitro continuously and produce an LPS-specific antibody continuously.

The thus established cell lines may be cultivated in vivo (e.g. nude mouse) or in vitro, followed by collection and purification of the antibody released in the culture medium or the ascites fluid to obtain the antibody in a large amount.

Production of the human monoclonal antibody of the invention comprises the following steps: (1) preparation of human B lymphocytes sensitized with an antigen; (2) establishment of monoclonal specific antibody-producing cell lines by immortalizing the cells as prepared in (1); (3) cultivation of the cell lines as established in (2); (4) purification of the monoclonal specific antibody from the culture as obtained in (3); and (5) production of an immunoglobulin preparation of high titer comprising the monoclonal specific antibody as purified in (4). Each of these steps will be hereinafter explained in detail.

Step (1):-

As the human B lymphocytes, there may be used human lymphocyte cells producing an antibody to LPS of P. aeruginosa and Lipid A of Gram-negative bacteria, which can be separated from a peripheral blood by the centrifugation using a lymphocyte separation liquid such as Lymphoprep® or Mono-Poly Resolving Medium® (Flow Lab.). There may be also used B lymphocytes originated from tissues or organs (e.g. lymphnode, spleen) extracted for the purpose of diagnosis or therapy of diseases, umbilical cord blood or the like. Those cells are desirable to be obtained from persons who were infected with P. aeruginosa in the past and whose cells are sensitized therein. Pertinent persons, from whom the cells may be obtained, can be chosen by previous measurement of the antibody titer in their sera. Alternatively, human B lymphocytes may be obtained from any person irrespective of his medical history in the past and mixed with inactivated P. aeruginosa, LPS originated from P. aeruginosa or Gram-negative bacteria which has a lipid A structure on its surface, preferably inactivated E. coli J5 or S. typhimurium Rc, Rd or Re mutant or LPS or Lipid A originated from those Gram-negative bacteria in vitro for sensitization. Namely, any of those antigens as inactivated may be added to B lymphocytes. Further, solutions containing lymphokines such as B cell proliferation factors and B cell differentiation factors (e.g. plant lectins such as poke-weed mitogen (PWM), bacterial body components such as Cowan I, human lymphocyte mixed culture, spleen, thymus or umbilical cord blood cell culture) may be added to human B lymphocytes for sensitization in vitro, followed by profileration and differentiation to give antibody-producing cells. The thus obtained human B lymphocytes have an antibody molecule at the cell surface can release a small amount of antibody for a certain limited period but they are not immortal.

Step (2):-

For changing the above sensitized human B lymphocytes to continuously proliferable immortal cell lines, there are fundamentally two processes, of which the first one comprises infection of B lymphocytes with EB virus by mixed cultivation of the sensitized human B lymphocytes with EB virus prepared from Marmoset cells B95-8, preferably using 2 to 10 $TD_{50}$ virus per cell. 96 well microplates were inoculated with cells of 0.5 to 3 x $10^4$ per well, and cultivation for transformation is effected using a usual medium (e.g. RPMI1640, Eagle's MEM) containing fetal bovine serum, bovine serum, horse serum or human serum in an amount of 2 to 20 % (v/v) in the presence of 5 to 10 % $CO_2$ at 32 to 37°C for 2 to 5 weeks, during which the medium is exchanged every half amounts with intervals of 2 to 4 days. If necessary, 1 to 2 $\mu g/ml$ of cyclosporin A and an antibiotic or antimicrobial agent for prevention of mycoplasma contamination may be added thereto. The thus transformed cells can be observed as colonies of 20 to 200 cells under an optical microscope 10 days or more after the infection and readily distinguished from non-transformed cells. The culture containing transformed cells as sufficiently proliferated in wells is subjected to screening for antibody titer with ELISA, the wells on which the antibody production is recognized are selected and the specificity to LPS is confirmed by the Western blotting method. Thereafter, the culture containing the transformed cell clusters is subjected to repeated aspiration and discharge with a pipette to break the clusters, diluted with the culture solution to make an appropriate cell concentration and inoculated onto a 96 well microplate to make 0.5 to 100 cells per well for cloning (limiting dilution method). Alternatively, cloning may be performed by the use of agarose (e.g. sea plaque agarose). For instance, the transformed cells (about $7 \times 10^4$ to $7 \times 10^5$) is inoculated into a culture plate (30 mmØ) of 0.36 to 0.4 % (w/v) agarose and cultivated at 37°C under 5 % $CO_2$ to obtain a colony proliferated from a single cell. On cloning, it is desirable to use mouse peritoneal cells, human umbilical cord blood lymphocytes or X ray-irradiated mouse spleen cells as the feeder layer. From the cloned cell lines, those having a high productivity of the specific antibody are selected by measurement of the antibody titers of the supernatants from the cultures of said cloned cell lines according to the ELISA method using the bacterial bodies of P. aeruginosa of 17 different serotypes and E. coli O-111:B4 and J5 as the solid phase antigen and further using LPS and lipid A originated from P. aeruginosa of 17 different serotypes, E. coli O-111:B4 and J5, S. typhimurium wild strain and Ra to Re mutants as the solid phase antigen. Cloning and selection as above are carried out repeatedly two or three times so as to establish a transformed cell line having a high proliferation rate and producing the specific antibody stably in a large amount.

The second process comprises subjecting the sensitized human B lymphocytes and myeloma cells to cell fusion in the presence of polyethylene glycol. The myeloma cells as used are hypoxanthine-guanine phosphoribosyl transferase (HGPRT)-deficient mutants (e.g. P3X63-Ag 8 (P3), P3X63-Ag 8.653) originated from mouse myeloma cell, HGPRT-deficient mutant originated from human myeloma cell U-266, HGPRT-deficient human-mouse heteromyeloma SHM-D33, etc. HGPRT-deficient mutants originated from human B lymphoblastoid cell are also usable.

As the polyethylene glycol (PEG), there may be used, for instance, PEG 1,000 to 6,000 in a concentration of 30 to 50 % (w/v). The fusion efficiency can be enhanced by incorporation of lectin, poly-L-lysine, dimethylsulfoxide, etc. thereto.

The fusion may be carried out, for instance, in the same manner as described in the Kohler et al. article (Nature, 256, 495 (1975)) wherein mouse cells are fused each other to obtain a hybridoma producing a mouse monoclonal antibody. For instance, the sensitized human B lymphocytes and HGPRT-deficient myeloma cells are mixed together in a proportion of 10 - 1 : 1, 30 to 50 % (w/v) PEG 4,000 is added portionwise thereto in 0.5 to 1 minute, and the resultant mixture is allowed to stand for 1 to 10 minutes. To the resulting mixture, 9 to 50 ml of a culture medium containing no serum are added in 5 to 10 minutes, and the culture medium is further added thereto to make a cell concentration of $10^5$ to $10^6$/ml. The cell suspension thus obtained is inoculated into a 96 well microplate at a rate of $2 \times 10^4$ to $2 \times 10^5$ cells per well. On the next day, the half amount is replaced by a hypoxanthine-aminopterinthymidine-containing medium (HAT medium) or a hypoxanthineazaserine-containing medium (HAz medium), and cultivation is effected at 32 to 37°C under 5 % $CO_2$ for about 2 to 3 weeks, during which the culture medium is replaced by a HAT medium for about 10 to 20 days and then by a hypoxanthine-thymidine-containing medium (HT medium) for about 3 to 5 days every half amounts at intervals of 3 days to obtain a proliferative colony, i.e. hybridoma. It is also possible to select a hybridoma by the combined use of metabolism inhibitors without using a HGPRT-deficient mutant. The antibody titer cf the culture medium of the hybridoma is measured by the ELISA method as above mentioned, the cell line producing the specific antibody having the above reaction specificity is selected, and the specificity to the polysaccharide is confirmed by the Western blotting method. Cloning is repeated two or three times by the limiting dilution method or the agarose method to obtain a stable cell line having a high rate proliferative property and a high specific antibody productivity.

In the first process as above explained, EB virus-transformed cells may be used in place of sensitized human B lymphocytes.

The cell lines as established from sensitized human B lymphocytes according to the EB virus transformation method or the cell fusion (hybridoma) method can be proliferated continuously and produce the specific antibody stably in a large amount.

Step (3):-

The thus established transformed cells or hybridomas (0.5 - $5 \times 10^5$ cells/ml) are incubated in a stationary state or under rotation at a usual culture medium for animal cells in a vessel such as an incubation flask or plate by the use of a $CO_2$ incubator at 32 to 37°C under 2 to 10 % $CO_2$. Particularly when incubation is made at a large scale, a jar fermenter, a hollow fiber system or the like designed for animal cells may be used. The usual culture medium may be, for instance, a medium containing 2 to 20 % serum of bovine fetus, calf, cow, horse, human or the like (e.g. RPMI1640, Eagle's MEM), a serum-free medium containing trace components required for the proliferation of cells (e.g. insulin, transferrin, ethanolamine, selenite, bovine albumin, lipid) or the like.

Step (4):-

Purification of the antibody may be carried out by conventional biochemical procedures (e.g. ammonium sulfate precipitation fractionation, ethanol precipitation fractionation, PEG fractionation, ion exchange chromatography, gel filtration, affinity chromatography, high speed liquid chromatography, electrophoresis). In the purification process, care should be taken for preventing the production of agglutination or the depression of antibody activity. For this purpose, human serum albumin (HSA) may be added in an amount of 0.05 to 2 %. Addition of amino acids such as glycine or alpha-alanine, especially basic amino acids such as lysine, arginine or histidine, saccharides such as glucose or mannitol, salts such as sodium chloride, etc. may be sometimes preferred. Agglutination tends to be produced, particularly in the case of an IgM antibody, and treatment with beta-propiolactone, acetic anhydride or the like is effective in prevention of such agglutination. In such case, intravenous administration will be made possible.

Step (5):-

The purified monoclonal antibody may be formulated into a biological preparation by a per se conventional procedure comprising, for instance, filtering through a membrane filter for removal of bacteria, admitting into sterilized vials with a stabilizer and freeze-drying.

The human monoclonal antibody preparation of the invention may comprise only one kind of human monoclonal antibody to LPS of P. aeruginosa for its use as a preventive or therap tic agent for infections with P. aeruginosa. Preferably, the preparation comprises additionally at least one kind of human monoclobnal antibody which can recognize the antigen-determinating site different in the molecular structure of LPS of P. aeruginosa and/or at least one kind of conventional human antibody which can recognize exotoxins, exoenzymes (e.g. elastase, protease), envelope proteins and endotoxins. The preparation may also comprise any human antibody to bacteria other than P. aeruginosa, virus, fungi, protozoa, cancer cells, etc. Furthermore, the human monoclonal antibody of the invention may be incorporated into a conventional human immunoglobulin preparation to make a high titer immunoglobulin preparation to LPS.

The human monoclonal antibody of the invention belongs mainly to Class IgG or IgM, but it is not limited thereto. The human monoclonal antibody has a binding property (binding activity) to P. aeruginosa of two or more kinds of serotypes, shows a bacteriocidal activity in the presence of a complement (complement bacteriocidal activity) and has an activity of accelerating the phagocytosis of neutrophile and macrophage for P. aeruginosa (opsonization). Experimental mouse infections with P. aeruginosa of two or more kinds of serotypes can be treated by administration of a single human monoclonal antibody of the invention.

The human monoclonal antibody of the invention has a specific binding property to the bacterial bodies of P. aeruginosa IID1001 (ATCC 27577), IID1006 (ATCC 27582), IID1020 and IID1015 among the standard strains for the serotype classification of P. aeruginosa. Further, it has a binding property to the lipid A of Gram-negative bacteria, and the biological activity such as lethal activity or Schwartzman reaction can be neutralized therewith.

The human monoclonal antibody can be bound to the lipopolysaccharides originated from E. coli Rc mutant J5 (ATCC 39355), S. minnesota rough type Rc, Rd and Re mutants and P. aeruginosa IID1001 (ATCC 27577) but not to those originated from E. coli smooth type strain O-111:B4, S. minesota smooth type wild strain, rough type Ra and Rb mutants and P. aeruginosa IID1002 (ATCC 27578) and PA 103.

As understood from the above, the human monoclonal antibody of the invention is different from known serotype- specific monoclonal antibody and known serotype sub-group cross-reacting monoclonal antibody. Since it can be bound to P. aeruginosa of two or more different serotypes, P. aeruginosa of all or almost all serotypes can be covered by a fewer number of monoclonal antibodies including the same. The human monoclonal antibody of the invention is thus highly valuable from the viewpoint of prevention and therapy of P. aeruginosa infections. In addition, it is useful for prevention and treatment of endotoxin shock, for which any effective means is presently not available.

The epitopes recognized by the human monoclonal antibody of the invention are the lipid A site and the polysaccharide site in LPS. The polysaccharide site to be recognized is limited to the one in certain serotype strains, i.e. Types A, F, G and M. It is therefore assumed that the structure common to the lipid A site and the polysaccharide site may be present.

For prevention and therapy of the infectious diseases with P. aeruginosa, the mixed infections with bacteria containing P. aeruginosa and the endotoxin shock due to Gram-negative bacteria, a human immunoglobulin preparation comprising the human monoclonal antibody may be administered to an adult patient in an amount of about 1 to 10 grams each time in case of the serious symptom or in an amount of about 0.2 to 5 grams each time in case of the non-serious symptom or the preventive purpose. The content of the human monoclonal antibody of the invention in the human immunoglobulin preparation is usually from about 0.5 to 500 mg, preferably from about 5 to 50 mg.

As stated above, the human monoclonal antibody of the invention has a high antibody titer to its antigen and shows an excellent therapeutic effect in the system of experimental mouse infections. Since it is a human-origin protein, any side effect (e.g. anaphylaxis) as seen on the administration of a heterogenic protein is not produced. Since it is produced from a certain specific cell line, a possibility of contamination with unknown biohazards is much less in comparison with conventional immunoglobulins produced from a human blood originated from a number of unknown persons.

The human monoclonal antibody of the invention is produced with a high antibody titer in vitro stably in a large amount, and its production process is more advantageous than conventional production processes using human blood in easy quality control.

The present invention will be hereinafter explained in details by way of examples, but it should be understood that this invention is not limited to those examples.

## Example 1

Establishment of human monoclonal antibody-producing cell lines by the EB virus transformation method:-

### (1) Preparation of the EB virus solution and measurement of its virus titer

EB virus-producing and releasing Marmoset cells B95-8 were suspended in an RPMI1640 medium containing 10 % fetal calf serum (FCS) at a rate of $6.5 \times 10^5$ cells/ml and incubated under a stationary condition in a culture flask T-75 (Corning #25110) at 37°C in 5 % $CO_2$ atmosphere for 4 days. The culture supernatant was collected and subjected to centrifugation by the aid of a low speed centrifuge (RS-20BH; Tommy Precision Ind. Co. Ltd.) at 2,000 rpm for 10 minutes. The supernatant was filtered through a 0.45 μ membrane filter (Millex SLHA 0250S). The filtrate was charged in serum tubes (MS-4505; Sumitomo Bakelite Co., Ltd.) and reserved at -80°C. On the use, the content was diluted appropriately and used for the experiment for transformation of human lymphocytes with EB virus.

The virus titer of the EB virus solution was determined according to the Moss et al method (J. General Virology, 17, 233 (1972)) using human peripheral blood lymphocytes (PBL) as the indicator cell. Namely, a suspension of human peripheral blood lymphocytes ($10^6$ cells/ml) in RPMI medium containing 15 % FCS was charged in a 96 well microplate (Sumitomo Bakelite) by 100 μl/well. To each well, the EB virus solution (20 ul) in 10 fold serial dilutions (within a range of $10^0$ to $10^7$) with the above medium was added, and incubation was conducted at 37°C in 5 % $CO_2$ atmosphere. The 1/3 volume of the medium was renewed every 3 days, and after 3 weeks, the presence of the transformed cells was examined by an optical microscope. The examination was made on 6 wells per each dilution, and the transformation rate was determined. The highest dilution required for transformation of 50 % cells was determined stochastically from the transformation rate at each dilution according to the Reed and Muench method, and the virus amount therein was calculated and taken as $1TD_{50}$, wherefrom the virus amount in the original sample was calculated back and designated by the $TD_{50}$ number. The EB virus solution having a virus amount of $10^5$ to $10^7$ $TD_{50}$/ml was thus obtained.

### (2) Measurement of anti-P. aeruginosa antibody titer according to the ELISA method

The antibody titer against P. aeruginosa surface antigen was measured as follows. P. aeruginosa was suspended in a phosphate buffer solution (pH 7.2; comprising NaCl (8 g/l), KCl (0.2 g/l), NaHPO₄.12H₂O (2.99 g/l and KH₂PO₄ (0.2 g/l)) (PBS) to give absorbance of 0.2 at a wavelength of 600 nm. The suspension

9

was charged in 96 well microplates ( Falcon #3912) at a rate of 50 μl/well, followed by centrifugation at 2,000 rpm for 15 minutes. 2 % Glutaraldehyde was added to each well at a rate of 50 μl/well to fix the bacterial body to the microplates. After removal of the bacterial solution from the microplates, 3 % PBS solution containing bovine serum albumin (BSA) was charged to the microplate at a rate of 120 μl/well and incubated at 37°C for 30 minutes for blocking of the unbound portion of the assay plate. The resulting microplate was used as the antigen-coated plate in the subsequent operation. When desired, storage of such microplate may be made at -20°C.

Prior to the assay, the microplate was washed with a 0.05 % Tween 20 containing PBS solution (PBST) three times. 1 % BSA containing PBST was charged into wells at a rate of 50 μl/well, and a sample (serum, ascitis fluid or culture supernatant), optionally diluted with a 1 % BSA containing PBST, was added thereto at a rate of 50 μl/well, followed by incubation at 37°C for 2 hours. The sample was removed from the plate, which was washed with PBST three times. Phosphatase-labeled, affinity chromatography-purified anti-human immunoglobulin antibody (Kirkegaard & Perry Lab. Inc.) (2nd antibody) diluted with 1 % BSA containing PBS solution in 500 to 1,000 folds was added to the microplate at a rate of 100 μl/well for incubation at 37°C for 2 hours. For measurement of the IgG antibody titer and the IgM antibody titer, there were respectively employed phosphatase-labeled anti-human IgG antibody and phosphatase-labeled anti-human IgM antibody. After removal of the 2nd antibody, the microplate was washed with PBST three times, and a substrate solution, i.e. an aqueous solution containing p-nitrophenylphosphoric acid disodium salt (3 mg) in 10 % diethanolamine buffer (pH, 9.1; 1 ml) containing $NaN_3$ (0.2 mg/1 ml) and $MgCl_2.6H_2O$ (0.1 mg/ml), was added to the microplate at a rate of 100 μl/well, followed by reaction at 37°C. After 45 minutes, 3N NaOH was added thereto at a rate of 20 μl/well to stop the reaction. The binding activity of the antibody ($OD_{405}$) was measured on Multiskan® (Titertek).

## (3) Preparation of lymphocytes from human peripheral blood

Human peripheral blood (100 ml) showing a high antibody titer to P. aeruginosa of plural serotypes was collected. Mono-Poly Resolving Medium® (15 ml; Flow Lab.) was charged into a centrifuge tube (50 ml volume; Sumitomo Bakelite), and the above human peripheral blood (20 ml) was slowly poured thereto, followed by centrifugation with a low speed centrifuge (BS-20BH, Tommy Precision Ind.) at 2,500 rpm (Roter-TS-7) and at room temperature for 15 minutes, whereby red blood cells and lymphocytes were separated. The portion containing lymphocytes was collected and washed with 10 % FCS containing RPMI1640 medium (hereinafter referred to as "medium") two times, followed by calculation of the cell numbers to obtain lymphocytes of $1.2 \times 10^8$.

## (4) Establishment of anti-P. aeruginosa LPS antibody-producing cell lines according to the EB virus transformation method

Human peripheral blood lymphocytes ($2 \times 10^7$) were suspended in the medium (2 ml), and the EB virus solution (10 ml; virus amount, $10^7$ $TD_{50}$/ml) was added thereto, followed by incubation at 37°C in 5 % $CO_2$ atmosphere for 2 hours.

The EB virus infected human lymphocytes were suspended in a Dulbecco's Modified Eagle's minimum essential medium (hereinafter referred to as "cell line establishing medium") comprising 15 % FCS, 10 % NCTC 109 tissue culture medium, penicillin (100 IU/ml), streptomycin (100 μg/ml), arginine (0.2 mg/ml), oxaloacetic acid (0.15 mg/ml), sodium pyruvate (0.05 mg/ml) and bovine insulin (0.2 U/ml) to make a concentration of about $2 \times 10^5$ cells/ml, and the suspension was admitted into a 24 well culture plate as previously charged with mouse peritoneal suspended cells (about $1 \times 10^5$ cells/well) at a rate of 0.5 ml/well. The lymphocytes were cultured at 37°C in 100 % humidity and 5 % $CO_2$ atmosphere, and the half amount of the medium was replaced by the fresh cell line establishing medium every 3 days from one week after the start of the incubation. After about one month, the cells were collected and used for cell fusion.

## (5) Cell fusion with EB virus-transformed cells

Mouse BALB/C myeloma cells originated from MOPC-21 line and deficient in HGPRT (P3X63-Ag8.653; ATCC CRL 1580) were subcultured in the cell line establishing medium, and $1 \times 10^7$ cells taken therefrom were washed with an Eagle's minimum essential medium three times. The EB virus-transformed cells (2x10⁷ cells) obtained in (4) were washed with an Eagle's minimum essential medium three times, mixed with myeloma cells (1 x 10⁷) in a centrifuge tube (Corning #25330) and subjected to centrifugation at 400 x g for 7 minutes. To the resulting pellet, PEG 4000 (1 ml; 45 % PBS solution, Merck) was added in 1 minute while rotating the centrifuge tube and stirring with a pipette, followed by allowing to stand at room temperature for 1 minute. An Eagle's minimum essential medium (9 ml) was added thereto in 5 minutes for dilution, and the resultant dilution was kept at 37°C for 1 hour. The pellet after centrifugation was suspensed in the cell line establishing medium (100 ml) and filled into 96 well microplates (Falcon #3040) at a rate of 1 x 10⁴ myeloma cells/well. Simultaneously, mouse BALB/C spleen cells as the feeder layer were added thereto at a rate of 3 x 10⁴ cells/well, followed by incubation at 37°C in 5 % CO₂ atmosphere. Every 2 or 3 days thereafter, the half volume of the medium was replaced by a selection medium. After cell fusion for about 2 weeks, the supernatants in the wells where proliferation was observed were subjected to examination on the production of antibodies to P. aeruginosa of different serotypes by the ELISA method. The fused cell (hybridoma) in the well showing a relatively high antibody titer was selected and subjected to further cultivation while cloning by the limiting dilution method. After about 2 weeks, the presence of antibodies to the common antigen determining site and binding to P. aeruginosa of plural serotypes was confirmed by the ELISA method on the culture supernatant of the clone. The clone was subjected to scale-up cultivation using a 24 well culture plate (MS-30240; Sumitomo Bakelite), a T-25 culture flask (#25100; Corning) and a T-75 culture flask (#25110; Corning). This clone was designated as "Hybridoma FKF-1F3" and deposited under accession number FERM P-9784 at Fermentation Research Institute, Agency of Industrial Science and Technology, located at Tsukuba, Ibaraki-ken, Japan. The human monoclonal antibody FKF-1F3 was IgM (μ, k).

Example 2

Analysis of the antigen recognized by the antibody FKF-1F3:-

(1) Preparation of LPS of P. aeruginosa

Preparation of LPS of P. aeruginosa was made in the same manner as in the Westphal et al. method ("Methods in Carbohydrate Chemistry", Academic Press, N.Y., Vol. 5, pages 83 to 91 (1965)). Namely, the bacterial body in wet (4 g) was treated with 45 % phenol warmed at 65 to 68°C, cooled below 10°C and subjected to centrifugation at 3,000 rpm for 15 minutes, whereby LPS was extracted to the aqueous layer. The aqueous layer was dialyzed to water for elimination of phenol and concentrated under reduced pressure to form LPS micelles, which were subjected to ultra-centrifugation to recover LPS. As the result, there were obtained each 2 mg of LPS of Type A standard strain IID1001, Type E strain PA103 and Type G standard strain IID1020. The bacterial body of P. aeruginosa as used was given from the Depository of the Medical Science Research Institute, Tokyo University, Japan. It is also available from Americal Type Culture Collection (ATCC) in the United States.

(2) Analysis of the antigen by the western blotting method

LPS of Types A and E of P. aeruginosa were subjected to electrophoresis on deoxycholic acid (DOC)/polyacrylamide gel according to the Komuro et al method (Resume of Reports for the 33rd Symposium on Toxins in Osaka, pages 94 to 99 (1986)), and the gel was dipped in transfer buffer (25 mM Tris-192mM glycine, pH 8.3, 20 % (v/v) methanol) at 4°C overnight and transferred to Durapore® membrane (Millipore) at room temperature, followed by incubation with a 2 % casein-containing PBS solution at room temperature for 1 hour for blocking. Further, incubation was made with a 0.1 % BSA solution and a 10 % FCS-containing TBS solution at room temperature for 1 hour. On the resultant blocking Durapore® membrane, aqueous solutions of the FKF-1F3 culture supernatant and of the human monoclonal antibody HI-006 (IgM) specifically recognizing Type E O-antigen as the control were incubated at 37°C for 1 hour and then at 4°C overnight. The Durapore® membrane was washed with 0.05 % Tween 20-containing TBS solution (pH, 8.0) five times and incubated with the 2nd antibody (peroxidase-labeled, anti-human immunoglobulin antibody diluted with 1 % BSA and 0.05 % Tween 20-containing PBS in 3,000 folds) at 37°C for 1 hour. Likewise, washing was made with 0.05 % Tween 20-containing PBS (pH, 8.0) five times, and coloring was effected with a coloring agent (PBS containing 0.5 mg/ml chloronaphthol, 20 % methanol and 0.08 % H₂O₂;

11

pH 7.5). As the result, a group of bands colored in ladder due to the reaction with the antibody FKF-1F3 was recognized at the lane of electrophoresis of P. aeruginosa Type A LPS, while no reaction took place at the lane of electrophoresis of P. aeruginosa Type E LPS. In case of HI-006 as the control, the bands in ladder were recognized at the lane of electrophoresis of Type E LPS, but no coloring was observed in any other lane.

## Example 3

Study on the binding spectrum of the antibody FKF-1F3 by the ELISA method:-

(1) Binding property of the antibody FKF-1F3 to the standard serotype strains of P. aeruginosa

In the same manner as in Example 1 (2), the binding property of the antibody FKF-1F3 to the standard serotype strains of P. aeruginosa was examined according to the ELISA method, of which the results are shown in Table 2. The strains as used were given from the Depository of the Medical Science Research Institute, Tokyo University, Japan and cultivated in heart infusion agar medium or a heart infusion broth medium.

Table 2

| Binding activity of the antibody FKF-1F3 to the standard serotype strains of P. aeruginosa according to the Japanese Committee' Classification | | |
|---|---|---|
| Serotype | Strain | Binding activity $(OD_{405})^*$ |
| A | IID 1001 | 0.6 |
| B | IID 1002 | 0 |
| B | IID 1007 | 0 |
| B | IID 1013 | 0 |
| B | IID 5004 | 0 |
| C | IID 1021 | 0 |
| D | IID 1004 | 0 |
| E | IID 1130 | 0 |
| F | IID 1006 | 0.4 |
| G | IID 1020 | 0.4 |
| H | IID 1009 | 0 |
| I | IID 1010 | 0 |
| J | IID 1011 | 0 |
| K | IID 1012 | 0 |
| L | IID 5141 | 0 |
| M | IID 5018 | 0.1 |
| M | IID 1015 | 0.3 |
| Note: | | |

*) Absorbance after coloring according to the ELISA method (45 minutes).

It was thus revealed that the antibody FKF-1F3 has a binding property specific to Types A, F, G and M.

(2) Binding property of the antibody FKF-1F3 to clicinally isolated strains

The binding property of the antibody FKF-1F3 onto Types A and G which are clinically isolated with high frequency was examined. For examination, 19 clinical isolates of Type A and 19 clinical isolates of Type G were used. As the result, the antibody was bound to 14 Type A clinical isolates (74 %) and 10 Type G clinical isolates (53 %) as shown in Tables 3 and 4.

Table 3

| Binding activity of the antibody FKF-1F3 to 19 serotype A clinical isolates | |
|---|---|
| Strain | Binding activity $(OD_{405})^*$ |
| SP6745 | 0.8 |
| SP6746 | 1.6 |
| SP6783 | 1.2 |
| SP6818 | 2.5 |
| SP6830 | 0.6 |
| SP6840 | 2.4 |
| SP6708a | 1.2 |
| SP9710 | 2.0 |
| SP9711 | 1.6 |
| SP9731 | 0.1 |
| SP9762 | 0.1 |
| SP9763 | 0.1 |
| SP9768 | 1.6 |
| SP9780 | 2.1 |
| SP10029 | 1.4 |
| SP10040 | 0.2 |
| SP10060 | 0.6 |
| SP10618 | 1.7 |
| SP10676 | 0.2 |
| Note: | |

*) Absorbance after coloring according to the ELISA method (after 45 minutes).

Table 4

| Binding activity of the antibody FKF-1F3 to 19 serotype G clinical isolates | |
|---|---|
| Strain | Binding activity $(OD_{405})^*$ |
| SP9704 | 1.7 |
| SP9709 | 1.6 |
| SP9712 | 0.1 |
| SP9714 | 1.4 |
| SP9717 | 0 |
| SP9718 | 0.8 |
| SP9738 | 1.7 |
| SP9785 | 1.1 |
| SP9743 | 1.8 |
| SP9792 | 0.1 |
| SP9755 | 0.1 |
| SP9761 | 0 |
| SP9767 | 1.3 |
| SP9772 | 0 |
| GN11187 | 0 |
| TL2378 | 1.7 |
| TL2424 | 0 |
| SP6788 | 1.3 |
| SP9728a | 0.3 |
| Note: | |

*) Absorbance after coloring according to ELISA method (after 45 minutes).

The binding activity of the antibody FKF-1F3 to the clinical isolates of P. aeruginosa Serotype M causing frequently chronic bronchoesophageal infections was also examined. The results are shown in Table 5, from which it is seen that the binding activity largely depends on the origin of the strains and said antibody is bound to the tested strains in 10 to 99 %.

Table 5

| Binding activity of the antibody FKF-1F3 to Serotype M clinical isolates | | |
|---|---|---|
| Strain | Place of isolation and/or year | Binding activity $(OD_{405})^*$ |
| SP9716 | Tokyo | 0.1 |
| SP9730 | 1984 | 0.1 |
| SP9744 | | 0.1 |
| SP9748 | | 0.4 |
| SP9749 | | 0.1 |
| SP9752 | | 0.1 |
| SP9775 | | 0 |
| SP10067 | | 1.1 |
| SP10675 | | 0.1 |
| SP6763 | Kyoto | 1.0 |
| SP6764 | 1984 | 0.9 |
| SP6765 | | 0.9 |
| SP6782 | | 1.2 |
| SP6794 | | 1.0 |
| GN6833 | | 0.6 |
| TL6852 | | 1.1 |
| TL6890 | | 0.1 |
| SP6892 | | 1.1 |
| SP6895 | | 0.8 |
| SP6908 | | 0.8 |
| Note: | | |

*) Absorbance after coloring according to the ELISA method (after 15 minutes)

## Example 4

Binding property of the antibody FKF-1F3 to E. coli J5:-

In the same manner as in Example 1 (2), the binding activity of the antibody FKF-1F3 to E. coli Rc type mutant J5 (ATCC 39355) was examined according to the ELISA method. The J5 strain was incubated in a heart infusion agar medium. The results are shown in Table 6, from which it is understood that the antibody FKF-1F3 is bound strongly to P. aeruginosa IID 1001 (Serotype A) as the positive control and weakly to E. coli J5.

Table 6

| Strain | Binding Activity $(OD_{405})$ |
|---|---|
| E. coli J5 | 0.3 |
| P. aeruginosa IID 1001 | 0.6 |
| (-) | 0 |

Example 5

Study on the binding specificity through a competitive system:-

Since it was confirmed that the antibody FKF-1F3 is bound to the bacterial bodies of P. aeruginosa IID 1001 (Serotype A) and P. aeruginosa IID 1002 (Serotype B), the binding specificity was examined through a competitive system, i.e. in the same manner as in Example 1 (2) by the ELISA method using a bacterial plate. As the competitive substance, there were used LPS (each 50 μg/ml) of P. aeruginosa IID 1001 (Serotype A), P. aeruginosa IID 1002 (Serotype B), P. aeruginosa 103 (Serotype E), P. aeruginosa IID 1020 (Serotype G), E. coli 0-111:B4 and J5. The results are shown in Table 7.

Table 7

| Antigen plate | Origin of competitive substance (LPS) | Binding activity ($OD_{405}$) |
|---|---|---|
| PA IID1001 strain | PA IID 1001 | 0.6 |
| | PA IID 1002 | 2.4 |
| | PA PA 103 | 2.4 |
| | PA IID 1020 | 1.9 |
| | EC 0-111:B4 | 2.5 |
| | EC J5 | 2.1 |
| | (-) | 2.3 |
| PA IID1020 strain | PA IID 1001 | 0.2 |
| | PA IID 1002 | 1.0 |
| | PA PA 103 | 0.9 |
| | PA IID 1020 | 0.7 |
| | EC 0-111:B4 | 1.0 |
| | EC J5 | 0.8 |
| | (-) | 0.9 |

In the bacterial body plates of P. aeruginosa IID 1001 and IID 1020, LPS originated from IID 1001 inhibited the binding of the antibody FKF-1F3 strongly, and LPS originated from IID 1020 showed a weak inhibitory activity. Other LPS showed no or only very weak activity.

Example 6

Binding property of the antibody FKF-1F3 to LPS originated from P. aeruginosa:-

P. aeruginosa origin LPS (1 μg/ml), phosphatidylcholine (5 μg/ml), cholesterol (2.5 μg/ml) were dissolved in ethanol, and the resultant solution was admitted into a 96 well plate at a rate of 10 μl/well, followed by allowing to stand at 37° C for 2 hours for drying. 3 % BSA-PBS solution (100 μl) was added thereto, followed by reaction at room temperature for 2 hours for blocking to obtain an antigen plate using LPS as the antigen (Kannagi et al.: "Application of Imunological Methods in Biomedical Science", Blackwell Scientific, Oxford, pages 117.1-117.20 (1986)).

ELISA was carried out in the same manner as in Example 1-(2) using 0.5 % BSA-PBS in place of PBST. The results are shown in Table 8.

Table 8

| Origin of LPS | Serotpye | Binding activity ($OD_{405}$) |
|---|---|---|
| P. aeruginosa A IID 1001 | A | 0.7 |
| P. aeruginosa A IID 1002 | B | 0.1 |
| P. aeruginosa PAO 1822 | B | 0.1 |
| P. aeruginosa PA 103 | E | 0.2 |
| P. aeruginosa IID 1020 | G | 0.3 |

The antibody FKF-1F3 was bound strongly to LPS originated from P. aeruginosa IID 1001 (Serotype A) and weakly to LPS originated from P. aeruginosa IID 1020 (Serotype G).

Example 7

Binding property of the antibody FKF-1F3 to LPS and lipid A originated from Salmonella:-

The binding property of the antibody FKF-1F3 to a series of LPS originated from Salmonella minnesota was investigated by the competitive reaction according to the ELISA method. The antibody FKF-1F3 was mixed with S. minnesota-origin LPS and lipid A (highly purified LPS kits; List Biological Laboratories, Inc.) as the competitive substances in concentrations of 2.5 $\mu$M and 25 $\mu$M, followed by incubation at 37°C for 1 hour. ELISA was carried out using a 96 well plate fixing the bacterial body of P. aeruginosa IID 1001 thereon for measurement of the inhibition rate. The results are shown in Table 9, from which it is understood that the antibody FKF-1F3 has a strong binding property to lipid A and LPS (rough type Rc, Rd and Re).

Table 9

| LPS Chemotype | Binding activity ($OD_{405}$) | |
|---|---|---|
| | 0.25 $\mu$M | 25 $\mu$M |
| LPS wild type | 0.24 | 0.29 |
| Ra | 0.27 | 0.25 |
| Rb | 0.24 | 0.22 |
| Rc | 0.22 | 0.07 |
| Rd | 0.17 | 0.04 |
| Re | 0.03 | 0 |
| Lipid A | 0.12 | 0.02 |
| (-) | 0.26 | 0.26 |
| Note: | | |
| The binding activity indicates the one after deduction of the value for blank (0.14). | | |

Example 8

Binding property of the FKF-1F3 to lipid A of Gram-negative bacteria:-

Investigation was carried out using E. coli-origin lipid A, S. typhimurium re-mutant-origin lipid A (Ribi Immunochem. Research Inc., Hamilton, MT) and S. minnesota R595-origin lipid A (List Biological Laboratories Inc.) in the same manner as in Example 7 according to the ELISA method. LPS and lipid A originated

17

from P. aeruginosa IID 1001 were prepared as follows: according to the Westphal et al. method, the water-soluble fraction obtained by extaction with 45 % hot phenol was treated with cetylmethylammonium bromide ("Cetavlon"; Nakarai Chemical) for elimination of nucleic acid, and the ethanol precipitate was used as a specimen of LPS (Methods Carbohydr. Chem., 5, 83-91 (1965)); LPS was hydrolyzed with 1 % acetic acid at 100°C for 90 minutes, and the chloroform extract was used as a specimen of lipid A (Eur. J. Biochem., 52, 331-343 (1975)).

In the same manner as in Example 7, ELISA was effected using a plate coated with S. minnesota R595-origin lipid A and and using lipid A as the competitive substance (50 μg/ml). The results are shown in Table 10, from which it is understood that the antibody FKF-1F3 has a binding property to lipid A originated from various Gram-negative bacteria.

Table 10

| Lipid A | Binding activity ($OD_{405}$) |
|---|---|
| P. aeruginosa IID 1001 | 0.25 |
| E. coli J5 | 0.18 |
| S. typhimurium Re mutant | 0.16 |
| S. minnesota R595 | 0.18 |
| (-) | 1.07 |

Example 9

Binding property of the antibody FKF-1F3 to LPS originated from P. aeruginosa:-

The binding property to LPS originated from P. aeruginosa IID 1001 was compared with that to LPS of E. coli and S. minnesota. ELISA was carried out in the same manner as in Example 8 using a plate coated with lipid A of S. minnesota R595 and using intact LPS and lipid A originated from P. aeruginosa IID 1001 (cf. Example 8) and smooth type LPS and lipid A originated from E. coli and S. minnesota as the competitive substances (50 μg/ml). The results are shown in Table 11, from which it is understood that the antibody can be bound to lipid A originated from any microorganism and smooth type LPS only originated from P. aeruginosa. The reason why the binding property to smooth type LPS is not seen is probably due to the micelle formation with LPS in aqueous solution, whereby the lipid A portion is not exposed on the steric hindrance of the polysaccharide portion. In case of P. aeruginosa, any antibody-binding site may be present in addition to lipid A.

Table 11

| Competitive substance | | Binding activity ($OD_{405}$) |
|---|---|---|
| P. aeruginosa IID 1001 | LPS | 0.16 |
| P. aeruginosa IID 1001 | Lipid A | 0.24 |
| E. coli 0-111:B4 | LPS | 1.02 |
| E. coli J5 | LPS | 0.18 |
| S. minnesota wild type | LPS | 1.07 |
| S. minnesota R595 | Lipid A | 0.20 |
| (-) | | 1.12 |

Example 10

EP 0 326 148 A1

Binding property of the antibody FKF-1F3 to chitin:-
Previously, Teng et al. succeeded in preparation of the human monoclonal antibody (IgM) to LPS of E. coli J5 (Proc. Natl. Acad. Sci., U.S.A., 82, 1790-1794 (1985)). In their article, it is indicated that the antibody has a binding property to chitin (N-acetylglucosamine homopolymer).

In this Example, investigation was made on the binding property of the antibody FKF-1F3 to chitin according to the ELISA method using a plate coated with S. minnesota R595-origin lipid A and using crab shell-origin chitin powder (Nakarai Chemical) as the competitive substance in the same manner as in Example 8. The results are shown in Table 12, from which it is understood that a cross-reaction is not seen between the antibody FKF-1F3 and chitin. Thus, the antibody FKF-1F3 is different from human anti-E. coli J5-origin LPS in epitope.

Table 12

| Competitive substance | Dose (μg) | Binding activity (OD$_{405}$) |
|---|---|---|
| Chitin | 5 | 1.05 |
| | 10 | 1.01 |
| S. minnesota R595-origin Lipid A | 5 | 0.33 |
| | 10 | 0.18 |
| (-) | - | 1.07 |

Example 11

Separation of the polysaccharide portion of LPS and analysis of the antigen:-

(1) Separation of the polysaccharide portion of LPS originated from P. aeruginosa IID 1001

According to the Wilkinson et al. method (Eur. J. Biochem., 52, 331 (1975)), the polysaccharide portion was separated from LPS originated from P. aeruginosa IID 1001. Namely, LPS (10 mg) was dissolved in 1 % acetic acid and hydrolyzed at 100°C for 90 minutes. The liberated lipid A was eliminated by fractionation with chloroform. The obtained water-soluble fraction was subjected to column chromatography (column size, 1 x 70 cm) using dextran ("Sephadex G50"; Pharmacia, Uppsala) equilibrated with 50 mM pyridine-acetate buffer (pH 5.5) for fractionation. The positions at which the polysaccharide, the semi-rough type core oligosaccharide and the rough type core oligosaccharide are eluted were detected by spectrophotometry. The neutral saccharide was detected by the phenol-sulfuric acid method Dubois et al.: Anal. Chem., 28, 350 (1956)), and the amino saccharide was hydrolyzed with 2 N sulfuric acid at 100°C for 2 hours and then detected by the MBTH (3-methyl-2-benzothiazolinone hydrazone hydrochloride) method (Tsuji et al.: Chem. Pharm. Bull., 17, 217 (1969)). The fractions of the polysaccharide, the semi-rough type core oligosaccharide and the rough type core oligosaccharide were recovered after freeze-drying.

(2) Binding property of the antibody FKF-1F3 to the polysaccharide fraction

Competition of the antibody FKF-1F3 with the polysaccharide, the semi-rough type core oligosaccharide and the rough type core oligosaccharide originated from P. aeruginosa IID 1001 as obtained in Example 12 (1) was investigated according to the ELISA method in the same manner as in Example 7. The amount of the competitive substance as used was the one corresponding to 20 nmol of the neutral saccharide according to the phenol-sulfuric acid method as mentioned in Example 12 (1). The results are shown in Table 13.

19

Table 13

| Competitive substance | Binding activity (OD$_{405}$) |
|---|---|
| Polysaccharide | 0.04 |
| Semi-rough type core oligosaccharide | 0.42 |
| Rough type core oligosaccharide | 0.39 |
| (-) | 0.39 |

From the above results and the western blotting in Example 2 (1), it may be said that the epitope as recognized by the antibody FKF-1F3 is present at the polysaccharide portion in addition to lipid A in case of certain strains of P. aeruginosa.


Example 12

Therapeutic effect of the antibody FKF-1F3 on the experimental P. aeruginosa infections in mice:-


(1) Therapeutic effect on the infections with P. aeruginosa (Setotype G)

ICR-slc mice (4 weeks old; male; 10 animals per group) were intraperitoneally inoculated with 1.2 x 10$^4$ or 6.2 x 10$^4$ cells of P. aeruginosa clinical isolate SP6788 (Serotype G). After one hour, the antibody FKF-1F3 (0.1 μg) was administered intraperitoneally. Judgement of the therapeutic effect was made on the survival rate after one week. The results are shown in Table 14, from which it is understood that all the animals are survived in the medicated groups, and the antibody FKF-1F3 is effective in treatment of the infections with P. aeruginosa (Serotype G).

Table 14

| Antibody | Dose | Survival rate (%) (inoculated amount, CFU/head) | |
|---|---|---|---|
| | | 1.2 x 10$^4$ | 6.2 x 10$^4$ |
| FKF-1F3 | 0.1 μg/head | 100 | 100 |
| None | - | 20 | 30 |


(2) Therapeutic effect on the infections with P. aeruginosa (Setotype A)

5 % Mutin ICR-slc mice (4 weeks old; male; 10 animals per group) were intraperitoneally inoculated with 3.7 x 10$^4$, 1.5 x 10$^5$ or 7.3 x 10$^5$ cells of P. aeruginosa clinical isolate SP6788 (Serotype A). After one hour, the antibody FKF-1F3 (0.1 μg) was administered intraperitoneally. Judgement of the therapeutic effect was made on the survival rate after one week. The results are shown in Table 15, from which it is understood that a significant survival rate is produced in the medicated groups even when the inoculated amount is so high as 1.5 x 10$^5$ cells, and the antibody FKF-1F3 is effective in treatment of the infections with P. aeruginosa (Serotype A).

Table 15

| Antibody | Dose | Survival rate (%) (inoculated amount, CFU/head) | | |
| --- | --- | --- | --- | --- |
| | | $3.7 \times 10^4$ | $1.5 \times 10^5$ | $7.3 \times 10^5$ |
| FKF-1F3 | 0.1 μg/head | 100 | 80 | 0 |
| BSA | 2 μg/head | 80 | 20 | 10 |
| Saline | - | 50 | 10 | 0 |

## Claims

1. A human monoclonal antibody, which has a specific binding property to the O-polysaccharides of the strains of Pseudomonas aeruginosa classified to two or more different serotypes under the Japanese Commitee's Classification and also a binding property to the lipid A originated from Gram-negative bacteria.

2. The antibody according to claim 1, wherein the serotypes are chosen from Types A, F, G and M.

3. The antibody according to claim 1 or 2, wherein the Gram-negative bacteria are chosen from Escherichia, Salmonella and Pseudomonas.

4. The antibody according to claim 3, which is reactive to the lipid A originated from Escherichia coli Rc mutant.

5. The antibody according to claim 4, which is reactive to the lipid A originated from Escherichia coli Rc mutant J5 (ATCC 39355).

6. The antibody according to any of claims 1 to 5, which is Igm.

7. A human monoclonal antibody FKF-1F3.

8. A preventive or therapeutic agent for bacterial infections, which comprises an effective amount of the antibody according to any of claims 1 to 7 as an active ingredient.

9. The agent according to claim 8, wherein the bacterial infections are caused by bacteria comprising Gram-negative bacteria.

10. The agent according to claim 9, wherein the bacterial infections are caused by bacteria comprising Pseudomonas aeruginosa.

11. A preventive or therapeutic agent for endotoxin shock caused by Gram-negative bacteria, which comprises an effective amount of the antibody according to any of claims 1 to 7 as an active ingredient.

12. An Epstein-Barr virus-transformed cell line which is capable of producing the antibody according to any of claims 1 to 7 and any descendant cell line originated therefrom.

13. A hybridoma obtained by cell fusion of a human B lymphocyte comprising an Epstein-Barr virus-transformed cell with a myeloma cell which is capable of producing the antibody according to any of claims 1 to 7 and any descendant cell line originated therefrom.

14. A hybridoma FKF-1F3 and any descendant cell line originated therefrom.

15. A process for producing a human monoclonal antibody which comprises subjecting the cell line according to any of claims 12 to 14 to cell culture and recovering the produced antibody from the culture supernatant.

16. The use of the human monoclonal antibody according to claims 1 to 7 for the preparation of a preventive or therapeutic against bacterial infections and endotoxin shock.

| | DOCUMENTS CONSIDERED TO BE RELEVANT | | EP 89101423.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | EP - A1 - 0 215 131 (TEIJIN LIMITED)<br><br>* Claims 3,5,7,9,11; abstract *<br><br>-- | 1,3,8-10,13 | A 61 K 39/40<br><br>A 61 K 39/104<br><br>A 61 K 39/108<br><br>A 61 K 39/112<br><br>C 12 P 21/00<br><br>C 12 N 5/00 |
| A | CHEMICAL ABSTRACTS, vol. 104, no. 5, February 3, 1986, Columbus, Ohio, USA<br><br>S.SAWADA et al. "A new common polysaccharid antigen of strains of Pseudomonas aeruginosa detected with a monoclonal antibody" page 437, Abstract-no. 32 782p<br><br>& J.Infect.Dis. 1985, 152(6), 1290-9<br><br>-- | 1,3, 13 | |
| P,A | FR - A1 - 2 606 421 (ROUSSEL-UCLAF)<br><br>* Claims 1,2 *<br><br>-- | 1,3 | **TECHNICAL FIELDS SEARCHED (Int. Cl.4)** |
| P,A | CHEMICAL ABSTRACTS, vol. 109, no. 7, August 15, 1988, Columbus, Ohio, USA<br><br>B.J.APPELMELK et al. "Production, characterization and biological effects of monoclonal antibodies to different parts of Gram-negative lipopolysaccharide core region" pages 512,513, Abstract-no. 52 858z<br><br>& Prog.Clin.Biol.Res. 1988, 272 (Bact. Endotoxins, 373-82)<br><br>-- | 1,3,5 | A 61 K 39/00<br><br>C 12 P 21/00<br><br>C 12 N 5/00<br><br>G 01 N 33/00 |

The present search report has been drawn up for all claims

| Place of search | Date of completion of the search | Examiner |
|---|---|---|
| VIENNA | 27-04-1989 | SCHNASS |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 101, no. 17, October 22, 1984, Columbus, Ohio, USA<br><br>L.M.MUTHARIA "Monoclonal antibodies specific for Escherichia coli J5 lipopolysaccharide: cross-reaction with other Gram-negative bacterial species"<br>page 532, Abstract-no. 149 444j<br><br>& Infect.Immun. 1984, 45(3), 631-6<br><br>-- | 1,3,5 | |
| A | EP - A1 - 0 183 876 (THE BOARD OF TRUSTEES OF THE LELAND STANFORD JUNIOR UNIVERSITY)<br><br>    * Claims 1-8 *<br><br>-- | 1,5,6, 11,13, 16 | |
| A | EP - A2 - 0 163 493 (GENETIC SYSTEMS CORPORATION)<br><br>    * Claims 1-6 *<br><br>---- | 1,3 | TECHNICAL FIELDS SEARCHED (Int. Cl.4) |

The present search report has been drawn up for all claims

| Place of search | Date of completion of the search | Examiner |
|---|---|---|
| VIENNA | 27-04-1989 | SCHNASS |